# EUROPEAN PATENT APPLICATION

(11) **EP 3 865 412 A1**
(43) Date of publication of application: **18.08.2021**
(21) Application number: 19870643.4
(22) Date of filing: 11.10.2019
(51) Int. Cl.: B65B 55/08, A61L 2/08

(54) **STERILIZATION DEVICE AND STERILIZATION METHOD**

(30) Priority: 11.10.2018 JP 2018192886
(71) Applicant: Suntory Holdings Limited, Osaka 530-8203 (JP)
(72) Inventor: TAKAHASHI Tokio, Soraku-gun, Kyoto 619-0284 (JP); ZAITSU Yu, Soraku-gun, Kyoto 619-0284 (JP); YOSHIHARA Kazuki, Kawasaki-shi, Kanagawa 211-0067 (JP); HIGASHIYAMA Kenichi, Soraku-gun, Kyoto 619-0284 (JP)
(74) Representative: Vossius & Partner Patentanwälte Rechtsanwälte mbB
(86) International application number: PCT/JP2019/040273
(87) International publication number: WO 2020/075846

(57) **Abstract**

A sterilizing device (1) according to the present invention is a sterilizing device (1) for emitting an electron beam to a target object (2) for sterilization of the target object (2), the sterilizing device (1) including: a lateral electron beam emitting section (3) configured to emit a first electron beam to the target object (2) from a lateral side relative to an axis direction connecting a mouth portion of the target object (2) with a bottom portion of the target object (2); and a mouth portion side electron beam emitting section (4) configured to emit a second electron beam to the target object (2) in the axis direction from outside on a side of the mouth portion, wherein a lateral dose and a mouth portion side dose differ from each other, the lateral dose being a dose of the first electron beam with which the target object (2) is irradiated at the lateral electron beam emitting section (3), the mouth portion side dose being a dose of the second electron beam with which the target object (2) is irradiated at the mouth portion side electron beam emitting section (4).

## Description

### Technical Field

The present invention relates to a sterilizing device and a sterilizing method.

### Background Art

A sterilizing process involving use of an electron beam has been in general use as means of sterilizing a target object such as containers such as plastic bottles, preforms of plastic bottles, and caps of plastic bottles.

Such a sterilizing process is, however, disadvantageous in that an electron beam cannot easily reach a thick portion of a container such as its mouth portion, with the result of possibly insufficient sterilization. Increasing the dose of irradiation for sufficient sterilization of a thick portion will unfortunately cause the container to be deformed due to heat generated by the electron beam irradiation. There has been proposed, as a method for solving the above issue, a method of additionally performing a sterilizing process involving use of a chemical agent (Patent Literature 1) or a method of emitting an electron beam from a nozzle inserted in a container (Patent Literature 2).

Containers sterilized with use of such a method ensure safety of products such as beverages, foods, and pharmaceutical agents.

### Citation List

### Patent Literature

Patent Literature 1
   Japanese Unexamined Patent Application Publication, Tokukai, No. 2015-217951
Patent Literature 2
   Japanese Unexamined Patent Application Publication, Tokukai, No. 2018-19805

### Summary of Invention

### Technical Problem

With techniques such as that disclosed in Patent Literature 1, removing the chemical agent completely after sterilization may be impossible or difficult, or cleaning a container for removal of the chemical agent may require a long time and a high cost. Techniques such as that disclosed in Patent Literature 2 require a process step of inserting and pulling out a nozzle through a narrow mouth portion of a container. This makes it difficult to increase the speed of the sterilizing process and requires a large-sized device. This may in turn increase the cost of production of, for example, the container itself as well as a beverage product using such a container.

The above circumstances have led to a demand for a device with a relatively simple structure for performing effective sterilization without use of a chemical agent.

### Solution to Problem

A sterilizing device according to the present invention is a sterilizing device for emitting an electron beam to a target object selected from a container, a container cap, and a container preform for sterilization of the target object, the sterilizing device including: a lateral electron beam emitting section configured to emit a first electron beam to the target object from a lateral side relative to an axis direction connecting a mouth portion of the target object with a bottom portion of the target object; and a mouth portion side electron beam emitting section configured to emit a second electron beam to the target object in the axis direction from outside on a side of the mouth portion, wherein a lateral dose and a mouth portion side dose differ from each other, the lateral dose being a dose of the first electron beam with which the target object is irradiated at the lateral electron beam emitting section, the mouth portion side dose being a dose of the second electron beam with which the target object is irradiated at the mouth portion side electron beam emitting section.

A sterilizing method according to the present invention is a sterilizing method for emitting an electron beam to a target object selected from a container, a container cap, and a container preform to sterilize the target object, the sterilizing method including: emitting a first electron beam to the target object from a lateral side relative to an axis direction connecting a mouth portion of the target object with a bottom portion of the target object; and emitting a second electron beam to the target object in the axis direction from outside on a side of the mouth portion, wherein a lateral dose and a mouth portion side dose differ from each other, the lateral dose being a dose of the first electron beam with which the target object is irradiated during the emitting of the first electron beam, the mouth portion side dose being a dose of the second electron beam with which the target object is irradiated during the emitting of the second electron beam.

Each of the above arrangements makes it possible to sterilize a target object sufficiently with use of an electron beam alone, without use of a chemical agent. Further, each of the above arrangements involves use of only elements for emitting an electron beam to a target object from outside thereof. This simplifies the device structure, increases the speed of the sterilizing process, and facilitates the device maintenance.

The description below deals with preferable embodiments of the present invention. The preferable embodiments described below as examples do not limit the scope of the present invention.

A sterilizing device as a preferable embodiment of the present invention is arranged such that for the sterilization of the target object, the lateral electron beam emitting section emits the first electron beam to the target object before the mouth portion side electron beam emitting section emits the second electron beam to the target object.

With this arrangement, the condition of the electron beam emission by the mouth portion side electron beam emitting section can be fine-tuned according to the degree of sterilization for the lateral electron beam emitting section. The above arrangement thus likely results in a good sterilizing effect.

A sterilizing device as a preferable embodiment of the present invention is arranged such that the mouth portion side dose is smaller than the lateral dose.

This arrangement makes it possible to use the mouth portion side electron beam emitting section as an auxiliary electron beam emitting section to sterilize a target object sufficiently without excessive electron beam emission.

A sterilizing device as a preferable embodiment of the present invention is arranged such that the mouth portion side dose is within a range of 3 kGy to 5 kGy.

This arrangement allows a target object to be sterilized sufficiently without being deformed due to electron beam irradiation.

A sterilizing device as a preferable embodiment of the present invention is arranged such that when the mouth portion side electron beam emitting section emits the second electron beam to the target object, an electron beam emitting source of the mouth portion side electron beam emitting section is apart from the target object by a separation distance whose minimum value is not more than 10 mm.

This arrangement minimizes that portion of the second electron beam which fails to contribute to sterilization of a target object as a result of collision with oxygen atoms. The above arrangement thus increases the efficiency of use of an electron beam and can reduce the number of electron beam emitting devices while keeping a necessary dose of electron beam irradiation.

A sterilizing device as a preferable embodiment of the present invention is arranged such that the sterilizing device has a logarithmic reduction value of not less than 6 with respect to the number of bacteria on an entire inner surface of the target object.

This arrangement allows the target object to be suitably used for products such as beverages, foods, and pharmaceutical agents.

Additional features and advantages of the present invention will be made clearer by the description of the exemplary and non-limiting embodiments below, which are described with reference to the drawings.

### Brief Description of Drawings

Fig. 1 is a diagram illustrating the flow of a process performed by a soft drink production facility including a sterilizing device according to the present invention.
Fig. 2 is a diagram illustrating a sterilizing device according to the present invention in its entirety.
Fig. 3 is an enlarged view of a lateral electron beam emitting section of a sterilizing device according to the present invention.
Fig. 4 is an enlarged view of a mouth portion side electron beam emitting section of a sterilizing device according to the present invention.

### Description of Embodiments

The description below deals with a sterilizing device as an embodiment of the present invention with reference to drawings. The present embodiment described below of the present invention is, as an example, a sterilizing device 1 that is for use in a soft drink production facility P and that is configured to sterilize a plastic bottle (which is an example of the "target object" and is an example container; hereinafter referred to simply as "bottle") 2 before the plastic bottle is filled with a soft drink.

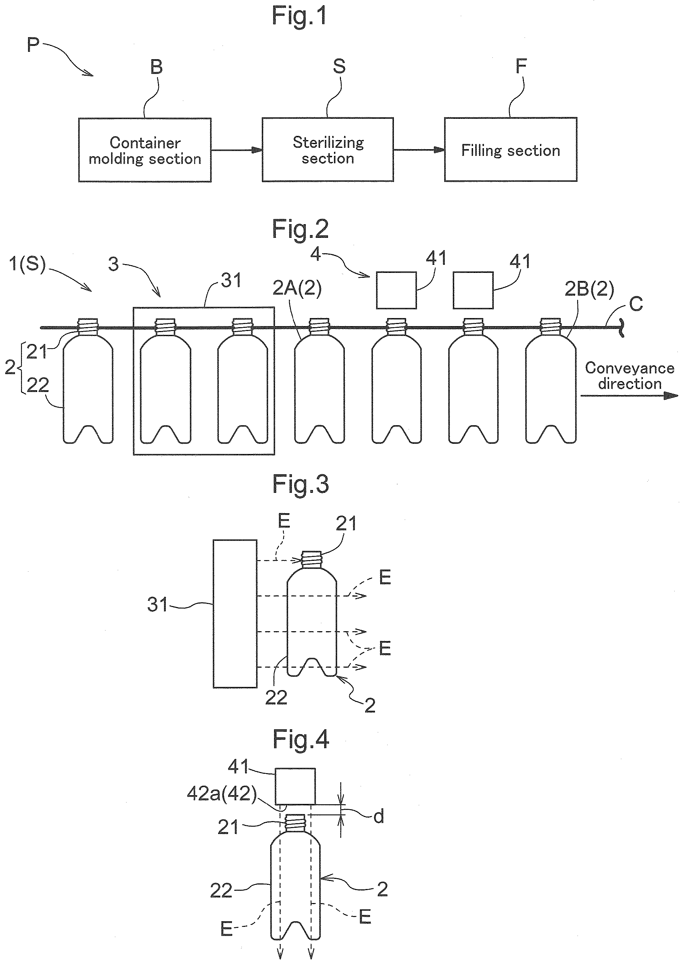

The production facility P includes a container molding section B configured to blow-mold a preform into a bottle 2, a sterilizing section S configured to sterilize the bottle 2 molded by the container molding section B, a filling section F configured to fill the bottle 2 sterilized by the sterilizing section S with a soft drink, and a conveying section C configured to convey the bottle 2 through the above sections (see Figs. 1 and 2). The sterilizing section S includes a sterilizing device 1 configured to emit an electron beam E to a bottle 2 being conveyed by the conveying section C for sterilization of the bottle 2. The conveying section C may be a publicly known device such as a star wheel or a conveyor belt. The present embodiment is arranged such that the bottle 2 is, while being conveyed by the conveying section C, supported by the conveying section C in such an orientation as to have a mouth portion 21 facing upward and a bottom portion facing downward.

The bottle 2 can be produced from a thermoplastic resin such as polyethylene, polypropylene, or polyethylene terephthalate as a main material, and molded integrally by a stretching and molding method such as biaxial stretching blow molding. The bottle 2 may have any capacity. The capacity may be approximately from 200 mL to 2 L such as 280 mL, 350 mL, or 500 mL, as of a common plastic bottle. The bottle 2 may be filled with any liquid. Examples include (i) beverages such as carbonated drinking water, soft drink, tea, fruit juice, coffee, cocoa, alcoholic beverage, milk beverage, and soup and (ii) liquid seasonings such as Worcester sauce and soy sauce.

### [Configuration of Sterilizing Device 1]

The description below deals with how the sterilizing device 1 is configured. The sterilizing device 1 includes (i) a lateral electron beam emitting section 3 configured to emit an electron beam E to a bottle 2 from a lateral side relative to an axis direction connecting the mouth portion 21 of the bottle 2 with the bottom portion of the bottle 2 (hereinafter referred to simply as "lateral side") and (ii) a mouth portion side electron beam emitting section 4 configured to emit an electron beam E to the bottle 2 in the axis direction from outside on the side of the mouth portion 21 (hereinafter referred to simply as "mouth portion side") (see Fig. 2). The lateral electron beam emitting section 3 and the mouth portion side electron beam emitting section 4 are arranged along the path of the conveying section C through which bottles 2 are conveyed. The lateral electron beam emitting section 3 and the mouth portion side electron beam emitting section 4 are each capable of emitting an electron beam E to bottles 2 being conveyed by the conveying section C, and do not require the conveyance to be stopped for the emission. The present embodiment is arranged such that bottles 2 are each conveyed in such an orientation that its mouth portion 21 faces above. The mouth portion side electron beam emitting section 4 is thus so positioned as to be capable of emitting an electron beam E from above bottles 2 being conveyed by the conveying section C.

The lateral electron beam emitting section 3 and mouth portion side electron beam emitting section 4 of the sterilizing device 1 are arranged along the path of the conveying section C such that the lateral electron beam emitting section 3 is present on the side of the entrance of the sterilizing section S, whereas the mouth portion side electron beam emitting section 4 is present on the side of the exit of the sterilizing section S. With this arrangement, a bottle 2 being conveyed inside the sterilizing section S is irradiated first with an electron beam emitted by the lateral electron beam emitting section 3 from the lateral side of the bottle 2 and then with an electron beam emitted by the mouth portion side electron beam emitting section 4 from outside on the mouth portion side of the bottle 2.

The lateral electron beam emitting section 3 includes a single high-voltage electron beam emitting device 31. The "high-voltage" portion indicates a voltage higher than that for each low-voltage electron beam emitting device 41 described below of the mouth portion side electron beam emitting section 4. The high-voltage electron beam emitting device 31 is so positioned as to be capable of emitting an electron beam E to a bottle 2 from a lateral side thereof over its entire dimension in the up-down direction (see Fig. 3).

The mouth portion side electron beam emitting section 4 includes two low-voltage electron beam emitting devices 41. The "low-voltage" portion indicates a voltage lower than that for the high-voltage electron beam emitting device 31 described above of the lateral electron beam emitting section 3. The low-voltage electron beam emitting devices 41 are each so positioned as to be capable of emitting an electron beam E to a bottle 2 from its mouth portion side (that is, from above the mouth portion 21). The low-voltage electron beam emitting devices 41 each include an electron beam emitting source 42 with a tip 42a. Each low-voltage electron beam emitting device 41 is so positioned that the tip 42a is apart from the mouth portion 21 of a bottle 2 by a separation distance d of 5 mm at a minimum (see Fig. 4).

### [Sterilization Method Involving Use of Sterilizing Device 1]

The description below deals with how the sterilizing device 1 is used to sterilize bottles 2.

Bottles 2 being conveyed by the conveying section C first enter the lateral electron beam emitting section 3 of the sterilizing section S. The high-voltage electron beam emitting device 31 of the lateral electron beam emitting section 3 emits an electron beam E to each bottle 2 from a lateral side thereof as the bottles 2 pass through the lateral electron beam emitting section 3.

After having been irradiated with an electron beam E at the lateral electron beam emitting section 3, the bottles 2 then enter the mouth portion side electron beam emitting section 4 of the sterilizing section S. The low-voltage electron beam emitting devices 41 of the mouth portion side electron beam emitting section 4 emit respective electron beams E to the bottles 2 from outside on the mouth portion side as the bottles 2 pass through the mouth portion side electron beam emitting section 4. The mouth portion side electron beam emitting section 4 causes each bottle 2 to be irradiated with an electron beam E in a dose of 4 kGy (mouth portion side dose), which is smaller than the dose of the electron beam E with which the bottle 2 is irradiated at the lateral electron beam emitting section 3 (lateral dose). The mouth portion side electron beam emitting section 4 has an accelerating voltage of 90 kV.

The mouth portion side dose is smaller than the lateral dose as above because the lateral electron beam emitting section 3 and the mouth portion side electron beam emitting section 4 have different roles: The lateral electron beam emitting section 3 serves as the main electron beam emitting section designed to sterilize the entire bottle 2 and thus configured to emit an electron beam E for a relatively large dose, whereas the mouth portion side electron beam emitting section 4 serves as an auxiliary electron beam emitting section designed to sterilize only the mouth portion of the bottle 2 and a portion therearound and thus configured to emit an electron beam E for a relatively small dose.

### [Evaluation of Degree of Sterilization]

The number of bacteria was determined on each of (i) a bottle 2 that had been irradiated with an electron beam E at the lateral electron beam emitting section 3 and that was to enter the mouth portion side electron beam emitting section 4 (hereinafter referred to as "bottle 2A") and (ii) a bottle 2 that had been irradiated with an electron beam E at the mouth portion side electron beam emitting section 4 (hereinafter referred to as "bottle 2B"). The number of bacteria thus determined was compared to the number of bacteria on a bottle 2 before being conveyed to the sterilizing section S to calculate the logarithmic reduction value (LRV) for evaluation of the degree of sterilization. The degree of sterilization was evaluated separately of the mouth portion 21 and body portion 22 of each bottle 2.

The number of bacteria can be determined with respect to any indicator bacterium selected in view of the sterilization strength desired. The evaluation herein was made of heat-resistant spores.

First, the body portion 22 of the bottle 2A showed a LRV of 6.0 as its degree of sterilization, indicating that the body portion 22 had been sterilized sufficiently. On the other hand, the mouth portion 21 of the bottle 2A showed a LRV of 3.6 as its degree of sterilization, indicating that the mouth portion 21 had been sterilized insufficiently. This is because the mouth portion 21 was thicker and did not allow an electron beam emitted from the lateral side to pass therethrough easily.

Next, the mouth portion 21 of the bottle 2B showed a LRV of 6.0 as its degree of sterilization, while the body portion of the bottle 2B also showed a LRV of 6.0 as its degree of sterilization. This indicates that while the mouth portion 21 of the bottle 2A (which had passed through the lateral electron beam emitting section 3 alone) was sterilized insufficiently as compared to its body portion 22, the mouth portion 21 of the bottle 2B had been sterilized sufficiently. As demonstrated above, a sufficient degree of sterilization (that is, with a LRV of not less than 6) for the entire bottle 2 cannot be achieved with use of the lateral electron beam emitting section 3 alone, but requires a combined use of the lateral electron beam emitting section 3 and the mouth portion side electron beam emitting section 4.

### [Effects of Sterilizing Device 1]

Suppose a case of sterilizing a container such as a bottle 2 with use of a sterilizing device that uses electron beam emission. Since such a container is typically long in the up-down direction, emitting an electron beam from the mouth portion side will not allow the container to be irradiated with the electron beam in its entirety, or even if the entire container is irradiated, the amount of irradiation will differ greatly between the mouth portion and the bottom portion, with the result of insufficient sterilization. In view of that, it has been a common practice to emit an electron beam from a lateral side of a container so that the entire container is irradiated with the electron beam uniformly. This practice, however, has involved the following issue: Since the mouth portion of a container is usually thicker than other portions to include a screw portion for attachment and detachment of a cap, the mouth portion will not allow an electron beam emitted from the lateral side to pass therethrough sufficiently, with the result of insufficient sterilization. Irradiating the mouth portion with the electron beam in a larger dose for sufficient sterilization will lead to another issue: Such an increased dose will cause the container to be deformed due to heat generated by the electron beam irradiation.

The sterilizing device 1, in view of the above issues, includes a lateral electron beam emitting section 3 and a mouth portion side electron beam emitting section 4: The former serves as the main electron beam emitting section responsible for overall sterilization of a bottle 2, while the latter serves as an auxiliary electron beam emitting section responsible for additional sterilization of the mouth portion 21 of the bottle 2, which mouth portion 21 cannot be sterilized sufficiently by the electron beam from the lateral electron beam emitting section 3 alone. With this arrangement, the mouth portion side electron beam emitting section 4 only needs to emit an electron beam E in a dose sufficient to compensate for the insufficiency in the degree of sterilization achieved with the electron beam emission by the lateral electron beam emitting section 3. This allows the mouth portion side dose to be smaller than the lateral dose. Further, the mouth portion side electron beam emitting section 4 emits an electron beam E to a bottle 2 from the mouth portion side, that is, from the side of the opening. This allows the electron beam E to reach the bottle 2 without being blocked by the mouth portion 21, that is, a thick portion made of thermoplastic resin. This also contributes to the small mouth portion side dose. Including a mouth portion side electron beam emitting section 4 as an auxiliary electron beam emitting section with a small mouth portion side dose as described above allows the entire bottle 2 to be sterilized sufficiently without deformation.

The sterilizing device 1 is also characteristic in that each low-voltage electron beam emitting device 41 of the mouth portion side electron beam emitting section 4 is so positioned that the tip 42a of the electron beam emitting source 42 is apart from the mouth portion 21 of a bottle 2 by a separation distance d of 5 mm when the tip 42a is closest to the mouth portion 21. The electron beam emitted by each electron beam emitting source 42 passes through an air layer before reaching a bottle 2. This causes a portion of the electron beam E to collide with, for example, oxygen molecules in the air and fail to reach the bottle 2. The sterilizing device 1 is, in view of that, configured such that such an air layer is thin, that is, the separation distance d is small. This reduces that portion of the electron beam E which collides with, for example, oxygen molecules and is lost, and allows the electron beam E, which is emitted by each low-voltage electron beam emitting device 41, to reach a bottle 2 efficiently for contribution to sterilization. This can reduce the number of low-voltage electron beam emitting devices 41 necessary for a mouth portion side dose of 4 kGy. This is preferable in terms of, for example, installation cost, installation area, and facility maintenance.

### [Other Embodiments]

The description below deals with sterilizing devices as other embodiments of the present invention. The arrangement disclosed for any embodiment below is combinable with the arrangement disclosed for any other embodiment unless such a combination causes any inconvenience.

The embodiment described above is an example arrangement in which a bottle 2 is irradiated first with an electron beam emitted by the lateral electron beam emitting section 3 from a lateral side of the bottle 2 and then with an electron beam emitted by the mouth portion side electron beam emitting section 4 from outside on the mouth portion side of the bottle 2. The present invention is, however, not limited to such an arrangement. The embodiment described above may be varied such that a bottle 2 is irradiated first with an electron beam emitted by the mouth portion side electron beam emitting section and then with an electron beam emitted by the lateral electron beam emitting section. The embodiment described above may alternatively be varied such that a bottle 2 is irradiated simultaneously with an electron beam emitted by the mouth portion side electron beam emitting section and an electron beam emitted by the lateral electron beam emitting section.

The embodiment described above is an example arrangement in which a bottle 2 to be sterilized is, while being conveyed by the conveying section C, supported by the conveying section C in such an orientation as to have a mouth portion facing upward and a bottom portion facing downward. The present invention is, however, not limited to such an arrangement. The embodiment described above may be varied such that a target object to be sterilized is, while being conveyed, so oriented as to have a mouth portion facing downward and a bottom portion facing upward. In this case, the mouth portion side electron beam emitting section is so positioned as to be capable of emitting an electron beam from below a target object being conveyed by the conveying section.

The embodiment described above is an example arrangement in which the mouth portion side dose is smaller than the lateral dose. The present invention is, however, not limited to such an arrangement. The embodiment described above may be varied such that the lateral dose is smaller than the mouth portion side dose. Note, however, that emitting a main electron beam to a target object in a direction traversing the shorter direction of the target object allows the entire target object to be irradiated with an electron beam sufficiently and can thus increase the degree of sterilization more easily.

The embodiment described above is an example arrangement in which the mouth portion side dose is 4 kGy. The present invention is, however, not limited to such an arrangement. A mouth portion side dose within the range of 3 kGy to 5 kGy easily allows the mouth portion 21 to have a high degree of sterilization. The mouth portion side dose is preferably selected as follows: Evaluate the degree of sterilization of a target object that has been irradiated with only an electron beam from the lateral electron beam emitting section; determine how insufficient the degree of sterilization is in comparison to a predetermined target degree of sterilization; and select a mouth portion side dose sufficient to compensate for the insufficiency in the degree of sterilization.

The embodiment described above is an example arrangement in which the mouth portion side electron beam emitting section has an accelerating voltage of 90 kV The present invention is, however, not limited to such an arrangement. An accelerating voltage within the range of 80 kV to 100 kV easily allows the mouth portion 21 to have a high degree of sterilization and is unlikely to require a large device. The accelerating voltage is, as with the mouth portion side dose, preferably selected as follows: Evaluate the degree of sterilization of a target object that has been irradiated with only an electron beam from the lateral electron beam emitting section; determine how insufficient the degree of sterilization is in comparison to a predetermined target degree of sterilization; and select an accelerating voltage sufficient to compensate for the insufficiency in the degree of sterilization.

The embodiment described above is an example arrangement in which each low-voltage electron beam emitting device 41 is so positioned that the tip 42a of the electron beam emitting source 42 is apart from the mouth portion 21 of a bottle 2 by a separation distance d of 5 mm when the tip 42a is closest to the mouth portion 21. The present invention is, however, not limited to such an arrangement. A separation distance d of not more than 10 mm is preferable as it allows an emitted electron beam to contribute to sterilization efficiently.

The embodiment described above is an example arrangement in which the sterilizing device 1 is used to sterilize a bottle 2 to be filled with a soft drink. The present invention is, however, not limited to such an arrangement. The sterilizing device 1 may alternatively be used to sterilize a container to be filled with, for example, an alcoholic beverage, seasoning, or a pharmaceutical agent.

The embodiment described above is an example arrangement in which the sterilizing device 1 is used to sterilize plastic bottles. The present invention is, however, not limited to such an arrangement. The sterilizing device 1 may alternatively be used to sterilize resin caps, aluminum caps, plastic bags, or plastic cups. In the case of sterilizing caps such as resin caps and aluminum caps, the present invention is applied such that the opening of a cap corresponds to the "mouth portion", whereas that portion of the cap which is opposite to the opening corresponds to the "bottom portion". In the case of sterilizing a container with no definite bottom portion such as plastic bags, the present invention is applied such that that portion of the container which is opposite to the mouth portion corresponds to the "bottom portion".

The embodiment described above is an example arrangement in which the sterilizing device 1 is used to sterilize a plastic bottle already molded in the shape of a container. The present invention may, however, alternatively be used to sterilize a preform of a plastic bottle. Such a preform can be sterilized with a smaller dose, allowing for a reduction in energy consumption and damage to the sterilization target.

It should be understood that with respect to other arrangements as well, the embodiments disclosed herein are illustrative in all respects and do not limit the scope of the present invention. Persons skilled in the art will readily understand that the present invention can be modified as appropriate without departing from the scope of the present invention. The present invention thus naturally covers in its scope any embodiment as modified without departing from the scope of the present invention.

### Industrial Applicability

The present invention is applicable to, for example, sterilization of a plastic bottle to be filled with a soft drink.

### Reference Signs List

- 1: Sterilizing device
- 2: Plastic bottle
- 21: Mouth portion
- 22: Body portion
- 3: Lateral electron beam emitting section
- 31: High-voltage electron beam emitting device
- 4: Mouth portion side electron beam emitting section
- 41: Low-voltage electron beam emitting device
- 42: Electron beam emitting source
- 42a: Tip of the electron beam emitting source
- E: Electron beam
- d: Separation distance between the tip 42a of the electron beam emitting source and the mouth portion 21
- P: Production facility
- B: Container molding section
- F: Filling section
- S: Sterilizing section
- C: Conveying section

## Claims

1. A sterilizing device for emitting an electron beam to a target object selected from a container, a container cap, and a container preform for sterilization of the target object,
the sterilizing device comprising:
a lateral electron beam emitting section configured to emit a first electron beam to the target object from a lateral side relative to an axis direction connecting a mouth portion of the target object with a bottom portion of the target object; and
a mouth portion side electron beam emitting section configured to emit a second electron beam to the target object in the axis direction from outside on a side of the mouth portion,
wherein a lateral dose and a mouth portion side dose differ from each other, the lateral dose being a dose of the first electron beam with which the target object is irradiated at the lateral electron beam emitting section, the mouth portion side dose being a dose of the second electron beam with which the target object is irradiated at the mouth portion side electron beam emitting section.

2. The sterilizing device according to claim 1, wherein
for the sterilization of the target object, the lateral electron beam emitting section emits the first electron beam to the target object before the mouth portion side electron beam emitting section emits the second electron beam to the target object.

3. The sterilizing device according to claim 1 or 2, wherein
the mouth portion side dose is smaller than the lateral dose.

4. The sterilizing device according to any one of claims 1 to 3, wherein
the mouth portion side dose is within a range of 3 kGy to 5 kGy.

5. The sterilizing device according to any one of claims 1 to 4, wherein
when the mouth portion side electron beam emitting section emits the second electron beam to the target object, an electron beam emitting source of the mouth portion side electron beam emitting section is apart from the target object by a separation distance whose minimum value is not more than 10 mm.

6. The sterilizing device according to any one of claims 1 to 5, wherein
the sterilizing device has a logarithmic reduction value of not less than 6 with respect to the number of bacteria on an entire inner surface of the target object.

7. A sterilizing method for emitting an electron beam to a target object selected from a container, a container cap, and a container preform to sterilize the target object,
the sterilizing method comprising:
emitting a first electron beam to the target object from a lateral side relative to an axis direction connecting a mouth portion of the target object with a bottom portion of the target object; and
emitting a second electron beam to the target object in the axis direction from outside on a side of the mouth portion,
wherein a lateral dose and a mouth portion side dose differ from each other, the lateral dose being a dose of the first electron beam with which the target object is irradiated during the emitting of the first electron beam, the mouth portion side dose being a dose of the second electron beam with which the target object is irradiated during the emitting of the second electron beam.
